# EUROPEAN PATENT SPECIFICATION

(11) **EP 4 183 320 B1**
(45) Date of publication and mention of the grant of the patent: **25.03.2026**
(21) Application number: 21306631.9
(22) Date of filing: 23.11.2021
(51) Int. Cl.: A61B 3/028, A61B 3/00, G02C 7/02

(54) **METHOD AND SYSTEM FOR COMPARING TWO OPHTHALMIC LENSES HAVING DIFFERENT OPTICAL DESIGNS**
VERFAHREN UND VORRICHTUNG ZUM VERGLEICH ZWEIER OPHTHALMISCHER LINSEN MIT UNTERSCHIEDLICHEM OPTISCHEM DESIGN
PROCÉDÉ ET APPAREIL POUR COMPARER DEUX LENTILLES OPHTALMIQUES AYANT DIFFÉRENTES CONCEPTIONS OPTIQUES

(43) Date of publication of application: 24.05.2023
(73) Proprietor: Essilor International, 94220 Charenton-Le-Pont (FR)
(72) Inventor: LE SAUX, Gilles, 75011 Paris (FR); GIRAUDET, Guillaume, 45000 ORLEANS (FR); MARIN, Gildas, 75012 Paris (FR)
(74) Representative: Jacobacci Coralis Harle

(56) References cited:
- US-A1- 2016 154 255
- US-A1- 2017 351 114

## Description

### TECHNICAL FIELD OF THE INVENTION

The invention relates to a method for comparing two ophthalmic lenses having different optical designs for providing a spherical and/or cylindrical power to correct the vision of an eye of a subject. It provides a method for allowing a subjective comparison of two ophthalmic lenses having different optical designs by an eye of a subject.

### BACKGROUND INFORMATION AND PRIOR ART

To determine a vision correction equipment adapted to a subject, the refractive properties of each eye of the subject are determined.

Today, these refractive properties may be obtained with two different methods: optical (objective) refraction and subjective refraction.

Optical objective refraction, which does not rely on the patient's response, can be obtained using instruments currently widely available such as skiascope, automatic refractometers and/or aberrometers.

Objective methods for comparing two ophthalmic lenses exist, one of them is presented in US2017/0351114.

These devices and methods however only provide information about the optical refractive error of the eye without taking into account the processing by the brain of the information provided by the eyes.

Therefore, eyeglasses are not usually manufactured based on optical objective refraction values of the eyes. These values are used as a starting point for a subjective refractive examination.

Subjective refraction relies on subject's perception to determine the optical correction giving the best possible visual acuity
Different devices and assessment protocols may be used for determining the subjective refraction values of the eyes of the subject.

The precision of traditional subjective refraction methods is 0.25D, which may result in an unsatisfactory final situation for the wearer. Recently developed devices use continuously variable lens modules associated with adapted assessment protocols allowing unprecedented precision of 0.01 diopter in determining the refraction values of the eyes of the subject.

However, the subject is not able to experience the benefit he may have with such preciseness in comparison with classical approaches.

Besides, different optical designs of the ophthalmic lens may be considered for any specific refraction previously determined.

The subject is usually not able to compare two ophthalmic lenses with different optical designs.

Current methods for comparing the effects of two ophthalmic lenses, such as the one presented in US2016/0154255, include the display, side by side, of two images, each image having a wavefront simulating the use of a different ophthalmic lens, for example each image having a different blur.

These methods are highly artificial as they do not place the subject observing said images in a real-life situation.

### SUMMARY OF THE INVENTION

Therefore, one object of the invention is to provide a new method allowing demonstrating to a subject the difference in visual perception between two ophthalmic lenses having different optical designs. The subject may then compare them and choose the most appropriate, that is the one providing the best visual performance and/or comfort.

The two ophthalmic lenses may be designed to provide the same refractive power or different refractive powers along a predetermined gaze direction through them.

The above object is achieved according to the invention by providing a method allowing a subjective comparison of two ophthalmic lenses having different optical designs by an eye of a subject, as described in claim 1.

This method is adapted to demonstrate the benefit of lenses determined with a high precision thanks to the use of new devices for determining the refraction values of the eye over lenses determined with a lower precision using classical devices. It is also adapted to compare different optical designs, especially two optical designs inducing different refraction errors or different optical aberrations on the periphery of said two ophthalmic lenses.

This method demonstrates the benefit of high precision refraction assessment and correction when the eye moves while looking through the lens, for example while the gaze direction of the subject moves from going through point A to going through point B of the lens. The method according to the invention is implemented after the determination of the refraction of the eyes, using the data obtained during this determination of the refraction. It does not comprise any step of determining a feature of the eye of the subject.

The two ophthalmic lenses are for example determined for providing a spherical and/or cylindrical power to correct the vision of the eye of the subject. The spherical and/or cylindrical power of this lens are determined based on the spherical and/or cylindrical refraction values of the eye of the subject.

The method according to the invention allows showing to the subject what he will experience looking through different parts of each of said two ophthalmic lenses. It is for example possible to experience looking through one or more peripheral parts of each of the two ophthalmic lenses.

The visual perception of the subject through a peripheral part of each of the two ophthalmic lenses may be quite different depending on the optical design of the ophthalmic lenses, even when the refraction power of the two ophthalmic lenses is the same at the optical center of each ophthalmic lens. This is especially the case when one of the two ophthalmic lenses is a regular lens with no aspherization and the other is an aspherical lens. It can also be the case when one of the lenses has a tint and the other has no tint, in other words is clear.

Other advantageous and non-limiting features of the method of the invention are listed in claims 2 to 14 o are as follows :
- said predetermined angle separating the two predetermined gaze directions is comprised between 10 and 50 degrees;
- said test lenses are obtained using a single test lens with variable optical power;
- in step a), a power map of each ophthalmic lenses is determined depending on gaze directions through the ophthalmic lens and said values of one or more optical features of the ophthalmic lens are determined based on this power map;
- said values of one or more optical features of each ophthalmic lens are determined taking into account average values of said fitting parameters or customized values of said fitting parameters determined for said subject.

The invention also relates to a system for allowing a subjective comparison by an eye of a subject of two ophthalmic lenses having different optical designs, as described in claim 15.

This system is used to implement the method according to the invention as described above.

### DETAILED DESCRIPTION OF EXAMPLE(S)

The following description with reference to the accompanying drawings will make it clear what the invention consists of and how it can be achieved. The invention is not limited to the embodiment/s illustrated in the drawings. Accordingly, it should be understood that where features mentioned in the claims are followed by reference signs, such signs are included solely for the purpose of enhancing the intelligibility of the claims and are in no way limiting on the scope of the claims.

In the following description and claims the word "to comprise" has an inclusive meaning.

On the appended drawings:
- figure 1 is a block schematic of a method according to the invention,
- figure 2 is a schematic view of an optometry system used to implement said method according to the invention,
- figure 3 is a schematic view of an ophthalmic lens showing iso curves of power error in a gaze direction diagram,
- figure 4 is a schematic view of an ophthalmic lens showing iso curves of unwanted astigmatism in a gaze direction diagram;
- figure 5 is a schematic profile view of an ophthalmic lens and the eye of the subject showing different gaze directions of the eye.

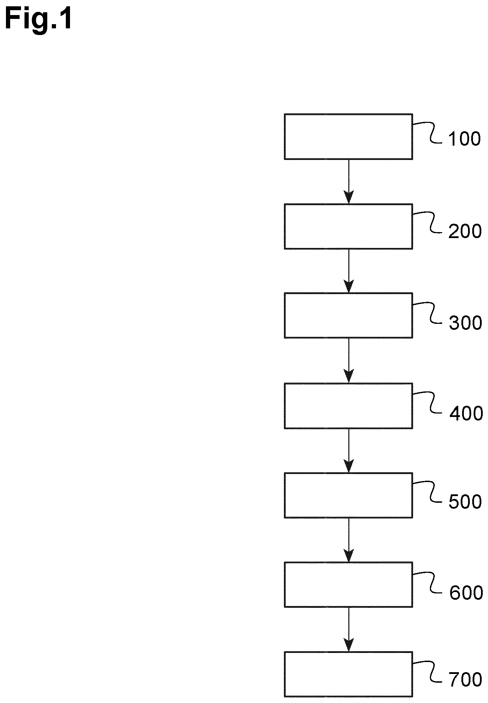

Figure 1 shows a block diagram of a method for comparing two ophthalmic lenses having different optical designs according to the invention.

According to the method of the invention, the following steps are performed:
a) determining (block 300 of figure 1) at least two values of one or more optical features of each ophthalmic lens of said two ophthalmic lenses, each value being determined along a predetermined gaze direction of an eye of a subject through said ophthalmic lens, the two predetermined gaze directions being separated by a predetermined angle,
b) for each ophthalmic lens of said two ophthalmic lenses, placing (block 400 of figure 1) successively in front of the corresponding eye of the subject test lenses, each of these test lenses exhibiting optical features having one of said at least two values of one or more optical features determined in step a) for this ophthalmic lens,
c) observing (block 500 of figure 1) a target through each of said test lenses,
d) comparing (block 600 of figure 1) the perception of this target through the test lenses corresponding to each ophthalmic lens of said two ophthalmic lenses.

Each of said two ophthalmic lenses is determined to improve the vision of said subject.

Each ophthalmic lens may be adapted to correct a visual defect of the eye of the subject, to improve the visual performance of the subject and/or improve the visual comfort of the subject. An example of the determination of the two ophthalmic lenses to be compared will be described later.

In a general manner, the two ophthalmic lenses may have different optical designs for providing the same refraction power to the eye of the subject, or they may have different optical designs for providing different refractive powers to the eye of the subject. The optical design of the ophthalmic lens is defined as the distribution of refractive power on the lens. In other words, different optical designs correspond to different power maps of the lenses. The optical design of a specific ophthalmic lens depends on the refractive power provided to the subject and on an optical design model used to determine the lens. Different optical designs may be obtained with either different refractive powers or different optical design models or both.

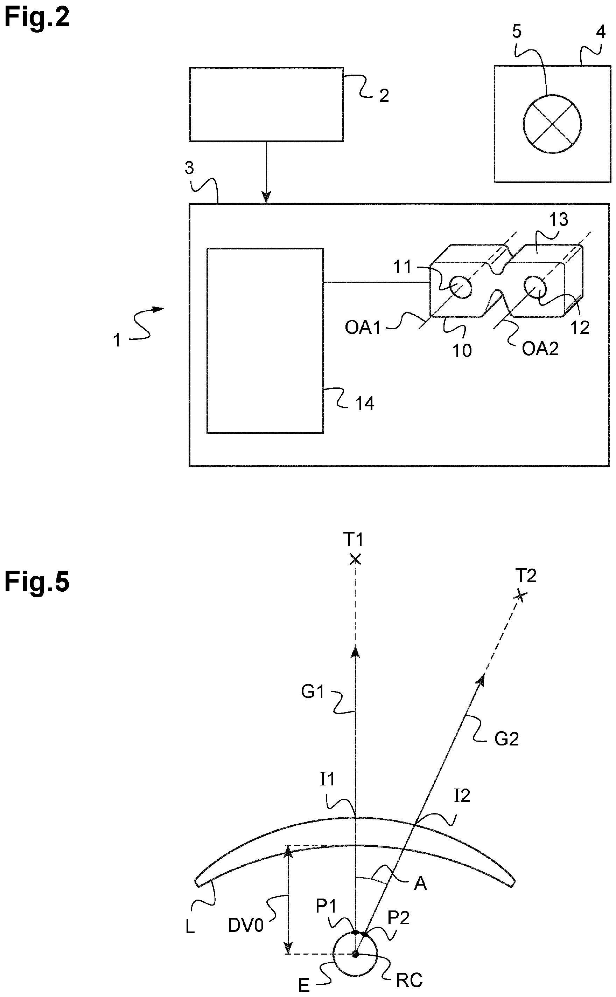

The method according to the invention may be performed thanks to an optometry system 1, the main elements of which are represented schematically on figure 2.

This optometry system 1 allows the eye of the subject to compare two ophthalmic lenses having different optical designs. It comprises:
- a device 2 programmed for determining at least two values of one or more optical features of each ophthalmic lens of said two ophthalmic lenses, each value being determined along a predetermined gaze direction of the eye of the subject through said ophthalmic lens, the two predetermined gaze directions being separated by a predetermined angle,
- a phoropter 3 controlled for providing test lenses, each of these test lenses exhibiting optical features having one of said at least two values of one or more optical features determined by said device,
- a target 5 placed in front of said test lenses provided by said phoropter.

### Step a)

In step a), at least two values of one or more optical features of each ophthalmic lens of said two ophthalmic lenses are determined, each value being determined along a predetermined gaze direction of an eye of a subject through said ophthalmic lens (block 300 of figure 1).

Said one or more optical features comprises one or more of the following: spherical power, cylindrical power and axis, prismatic power and axis, transmittance of the lens at a wavelength or within a predetermined range of wavelengths.

The spherical power, cylindrical power and axis, prismatic power of the lens are optical dioptric features of the lens. The transmittance of the lens along a specific gaze direction at a specific wavelength or within a specific wavelength range corresponds to the ratio between the intensity or luminance of the light at said specific wavelength or within said specific wavelength range incident on the lens to the intensity or luminance of the light at said specific wavelength or within said specific wavelength range transmitted by said lens.

As shown schematically on figure 5, the gaze direction G1, G2 of the eye E may be defined as the straight line passing through the center of rotation CR of the eye E, the center of the pupil P1, P2 of the eye E, a point I1, I2 of the front face of the lens L and the target T1, T2 that the subject is looking at, if any.

Figure 5 is a schematic view showing two positions of the pupil P1, P2 and therefore two gaze directions G1, G2, overlapped.

The two predetermined gaze directions G1, G2 are separated by a predetermined angle A.

Said device 2 (figure 2) comprises a computer having one or more processors and one or more memories programmed for calculating said at least two values of one or more optical features of each ophthalmic lens of said two ophthalmic lenses and storing said values in said one or more memories.

Said optical features may be calculated using average wearing conditions or using the specific fitting parameters of the subject. Average wearing conditions may comprise statistical average conditions for a population, standard conditions, any predetermined conditions, or usual conditions for the subject.

In other words, the distance DVO between the center of rotation RC of the eye E and the lens L, for example its back face, used in this calculation may be an average distance, not customized for said subject, or may be a specific distance customized for the subject.

The specific distance DVO between the center of rotation RC of the eye E and the lens L customized for the subject may be measured, for example based on a profile image of the subject wearing eyeglasses or determined based on morphological parameters of the subject.

An average or specific position of the center of rotation CR of the eye of the subject relative to the ophthalmic lens L may be determined. All the possible gaze directions used by the subject when the subject's gaze scans the ophthalmic lens between two points of the back or front faces of the lens may be calculated. The two points may comprise any two points of the ophthalmic lens.

The two points may comprise the optical center of the lens and any other point, in particular a point having an eccentricity above 5 degrees compared to the optical center.

The other point may also be a peripheral point, that is to say a point closer to the edge of the lens than to the center of the lens.

Alternatively, the two points may comprise two points having an eccentricity over 5 degrees compared to the optical center or two peripheral points.

One of the two points could correspond to a fixation cross of each of said two ophthalmic lenses or a center of the near vision zone for example.

It is especially interesting to compare the values of the one or more optical features for two gaze directions exhibiting different eccentricities as the errors and/or aberrations of each ophthalmic lens often increases radially from a given point of the lens towards the edge of the lens.

The errors and/or aberrations of lenses depends on the absolute value of the refractive power of the lens and on the optical design model of the lens. For example, a lens determined with a specific absolute value of a dioptric power and an aspherized optical design model globally exhibits a smaller radial increase in errors and/or aberrations than a lens having the same specific absolute value of dioptric power with a non aspherized optical design model.

A lens determined with a specific optical design model (spheric or aspherized) and an absolute value of dioptric power globally exhibits a smaller radial increase in errors and/or aberrations than a lens having the same specific optical design model and a higher absolute value of dioptric power.

For example, optical features of each ophthalmic lens such as spherical power, cylindrical power and axis, horizontal or vertical prismatic power between the two eyes or transmittance may be calculated for a first gaze direction G1 of the subject going through the center of the lens and for a second gaze direction G2 of the subject going through a peripheral point of the back or front face of the lens having an eccentricity of 40°.

Horizontal and vertical prismatic power correspond to the decomposition of the prismatic power in horizontal and vertical components.

In the following description, the eccentricity of a point of the back or front face of the lens is defined as the value of the angle A between the gaze direction G1 of the subject going through the center of the lens and the gaze direction of the subject going through this point.

In a binocular embodiment of the method according to the invention, two couples of ophthalmic lenses are to be compared, each lens of the two couples of lenses being intended to be placed in front of one of the eyes of the subject. An optical feature of the couple of lenses may be determined. It may comprise the prismatic difference between the two lenses of said couple. The prismatic difference may be defined as the difference between the prismatic power of each lens of said couple of lenses along an identical gaze direction through each lens.

In the case where the optical feature comprises a transmittance of the ophthalmic lens at a wavelength or within a wavelength range, it is then possible to compare lenses without tint and tinted lenses or lenses having different tints as tint may influence the subject's eye refraction.

It is then possible to compare the perception of the subject through a first ophthalmic lens having a tint and a spherical and/or cylindrical and/or prismatic power for correction or improvement of the subject's visual performance with the perception of this subject through a second ophthalmic lens having the same spherical and/or cylindrical and/or prismatic power for correction or improvement of the subject's visual performance with no tint. It is also possible to compare the perception of the subject through a first ophthalmic lens having a first tint and a spherical and/or cylindrical and/or prismatic power for correction or improvement of the subject's visual performance with the perception of this subject through a second ophthalmic lens having the same spherical and/or cylindrical and/or prismatic power for correction or improvement of the subject's visual performance with a second tint different from the first one.

Of course, one may also consider comparing the perception of the subject through a first ophthalmic lens having a tint and a first spherical and/or cylindrical and/or prismatic power for correction or improvement of the subject's visual performance with the perception of this subject through a second ophthalmic lens having a second different spherical and/or cylindrical and/or prismatic power for correction or improvement of the subject's visual performance or no spherical and/or cylindrical and/or prismatic power with the same tint.

In practice, in order to provide test lenses with or without tint or different tints, the phoropter 3 may comprise different filters adapted to be placed between the eye of the subject and the target 5. Each filter may allow the phoropter 3 adapting the chromaticity or the light transmission level of the test lens. It may also comprise an active filter allowing adapting chromaticity and/or light transmission level.

In practice, said values of one or more optical features of each ophthalmic lens may be determined taking into account the fitting parameters of the ophthalmic lens on the head of the subject.

In this case, the values of the optical features of the lens along said predetermined gaze direction takes into account the position and orientation of the lens relative to the eye of the subject.

Said values of one or more optical features of each ophthalmic lens may be determined taking into account average values of said fitting parameters or customized values of said fitting parameters determined for said subject.

Said fitting parameters may comprise for example interpupillary distance or half interpupillary distance and fitting height, that is to say the vertical distance between the center of the pupil and the bottom edge of the lens in worn conditions.

The average value of each fitting parameter may be determined for a population of subjects, for example subjects within a predetermined range of age or refraction value. The customized value of each fitting parameter may be measured on the subject with or without his eyeglasses and/or calculated based on measurements performed on the subject with or without his eyeglasses.

Said values of one or more optical features of each ophthalmic lens may also be determined taking into account the sensitivity of the subject to a variation of one or more of said optical feature. The sensitivity of the subject to a variation may be determined for example thanks to the method described in document WO2020/016398. The sensitivity of the subject to a variation of one optical feature is defined as the smallest variation of the optical feature that may be perceived by the subject. A global sensitivity may be defined for one or both eyes of the subject relative to the variation of one or more optical features of the ophthalmic lens or lenses.

For example, the predetermined gaze directions along which said two values of one or more optical features of each ophthalmic lens of said two ophthalmic lenses are determined may be determined taking into account the sensitivity of the subject.

The two gaze directions each going through a different point of the ophthalmic lens, the distance between these two points of the lens may be determined taking into account the sensitivity of the subject.

In practice, the two gaze directions, and hence, the positions of the two points on the lens, are determined so that the difference between the two values of said one or more optical features of the lens will be perceived by the subject, taking into account his sensitivity. In other words, the two gaze directions are determined so that the difference between the two values of a given optical feature is higher than the sensitivity of the subject to a variation of this optical feature.

As a consequence, for a subject having a higher sensitivity than another subject, the distance between the two points or the angle between the two gaze directions may be smaller than for this other subject.

In a possible example, the benefit of having an aspherized ophthalmic lens instead of a regular spherical lens is to be shown to two subjects having both a refraction of +4.00D. The first subject exhibits a higher sensitivity to refractive changes: the two gaze directions determined go through the optical center and another point with 20° eccentricity. For the second subject, who has a lower sensitivity than the first one, the two gaze directions determined go through the optical center and another point with 30° or 35° eccentricity. A larger distance between the two points is necessary in the case of the second subject to show the difference between both lenses.

In a preferred embodiment, the predetermined angle between the two predetermined gaze directions considered in step a) of the method is determined taking into account a refraction value of the eye of the subject and/or said sensitivity of the eye of the subject to a variation of said optical features of the ophthalmic lens.

As mentioned above, the difference between the two values of a given optical feature should be higher than the sensitivity of the subject to a variation of this optical feature for the subject to perceive them.

As a consequence, the angle between the two predetermined gaze directions determined increases when the sensitivity of the subject decreases and the angle between the two predetermined gaze directions determined decreases when the refraction absolute value increases.

Alternatively, the predetermined angle between the two predetermined gaze directions considered in step a) of the method can be set arbitrarily.

For example, said predetermined angle separating the two predetermined gaze directions is comprised between 10 and 50 degrees.

As mentioned, in a preferred embodiment, one of said predetermined gaze direction goes through the optical center of the ophthalmic lens, and the other predetermined gaze direction goes through an off-center point separated from the optical center by said predetermined angle.

Alternatively, in an embodiment, in step a), a power map of each ophthalmic lenses is determined depending on gaze directions through the ophthalmic lens and said values of one or more optical features of the ophthalmic lens are determined based on this power map. The power map comprises the values of the optical features of the ophthalmic lens for each gaze direction through the lens of a predetermined set of gaze directions.

The power map may also comprise said values of one or more optical features of the ophthalmic lens for gaze directions defining a continuous movement of the eye of the subject between two points of one of the back and front face of the lens.

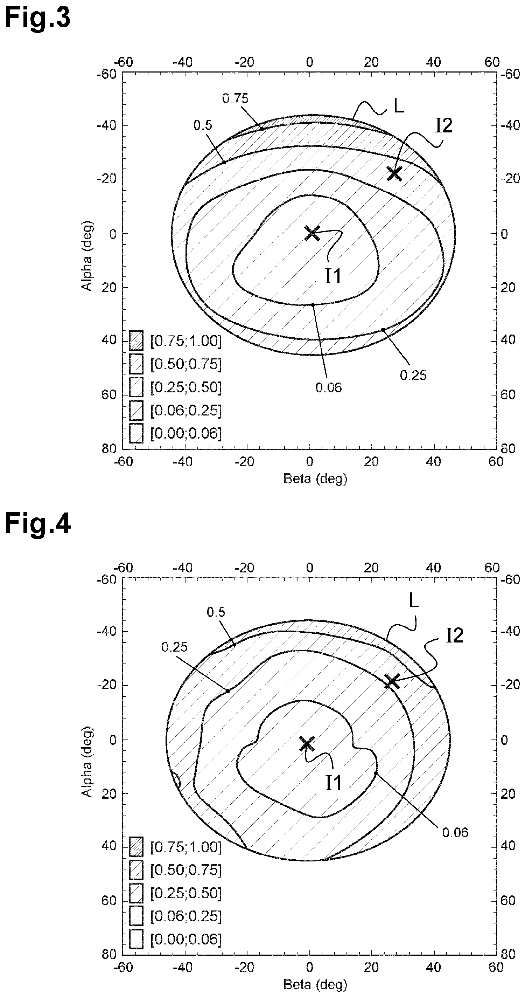

Such power maps are for example shown on figures 3 and 4. These figures show the power maps for an optical design computed by said device 2 based on the refraction values of the subject. The refraction values are for example the following spherical power S = - 4D, cylindrical power C= - 2D and Acyl = 0°. This optical design aims at maximizing the zone of the lens providing an error on spherical and cylindrical power below a threshold value. The central zones of the lens represented schematically on figures 3 and 4 are the ones with lower error. The error then increases radially towards the edge of the lens.

As represented schematically on these figures, the points I1 and I2 are located in zones exhibiting different errors. The optical features of the lens for gaze directions going through these points are then different.

Different gaze directions may then be used to simulate different visual situation such as straight-ahead vision, convergent gaze for near vision task, peripheral vision on the left or right side, to the top or bottom of the lens, smooth pursuit as described hereafter, ocular saccades, going from far to near vision and from near to far vision. The two ophthalmic lenses could then be compared in each of these situations.

### Step b)

In step b), the subject is provided by the system according to the invention with test lenses exhibiting optical features having one of said at least two values of one or more optical features determined in step a) for each ophthalmic lens of said two ophthalmic lenses (block 400 of figure 1).

For each ophthalmic lens of said two ophthalmic lenses, at least two test lenses are placed (block 400 of figure 1) successively in front of the corresponding eye of the subject test lenses, each of these test lenses exhibiting optical features having one of said at least two values of one or more optical features determined in step a) for the corresponding ophthalmic lens.

More precisely, in step a), said at least two predetermined gaze directions of the eye of the subject through said ophthalmic lens may comprise a predetermined discrete number of gaze directions and in step b), a corresponding predetermined discrete number of test lenses is placed in front of the eye of the subject.

Said predetermined number of gaze directions is for example comprised between 2 and 10 gaze directions.

It is possible to use a classical phoropter placing corresponding predetermined number of different test lenses having different optical features each corresponding to the optical features of one of said two ophthalmic lenses along one of the predetermined gaze directions.

However, in a preferred embodiment, said test lenses are obtained by modifying the optical power of a single test lens with variable optical power in order for said single test lens to exhibit successively each value of said two values of one or more optical features determined in step a). The main features of the corresponding phoropter 3 are described hereafter.

The system according to the invention controls the variation of the optical power of the test lens with variable optical power in order for it to exhibit the optical features determined in step a).

It is then possible to control the test lens with variable optical power for it to exhibit discrete successive values of the optical features, or to exhibit continuously changing values of said optical features.

Step b) may therefore be performed in two different ways.

In a static embodiment of the method, performed with a classical phoropter or with a phoropter equipped with a test lens having a variable power, discrete successive values of the optical features of each of the two ophthalmic lenses are provided to the eye of the subject for comparison.

For example, for each of said two ophthalmic lenses, said test lens with variable power is set to exhibit successively the optical features of:
- the first of said two ophthalmic lenses with a first gaze direction going through the center of this first lens;
- the first of said two ophthalmic lenses with a second gaze direction going through a predetermined peripheral point and oriented à 40° from the first gaze direction;
- the second of said two ophthalmic lenses with the first gaze direction going through the center of this second lens;
- the second of said two ophthalmic lenses with the second gaze direction going through said predetermined peripheral point and oriented à 40° from the first gaze direction.

This static embodiment simulates ocular saccades of the subject from the center of each of said two ophthalmic lenses to the peripheral point, for example along a horizontal direction.

The time elapsed during the change between two successive values of the optical feature is preferably short, for example below or equal to 500 milliseconds, preferably below or equal to 250 milliseconds.

The parameters of this static simulation, such as the time elapsed between two successive values of the optical features or the angle A may be predetermined and the same for every subject, or may be customized for each subject, for example depending on the refraction of the eye of the subject and/or on the sensitivity of the eye of the subject to the variation in the optical features of the lenses.

In a dynamic embodiment of the method, performed with a phoropter equipped with a test lens having a variable power, continuously changing values of the optical features of each of the two ophthalmic lenses are successively provided to the eye of the subject for comparison.

For example, for each of said two ophthalmic lenses, said test lens with variable power is set to exhibit optical features continuously changing as if the eye of the subject was following a path between the first and second gaze directions, for example between
- the first gaze direction going through the center and the second gaze direction going through a predetermined peripheral point and oriented à 40° from the first gaze direction for the first of said two ophthalmic lenses; and
- the first gaze direction going through the center and the second gaze direction going through a predetermined peripheral point and oriented à 40° from the first gaze direction for the second of said two ophthalmic lenses.

This dynamic embodiment simulates a smooth pursuit of a moving target by the subject from a first gaze direction to a second one.

In this dynamic embodiment, in step a):
- said at least two predetermined gaze directions of the eye of the subject through said ophthalmic lens comprise a continuous set of gaze directions corresponding to the gaze directions of the eye of the subject moving continuously between said two predetermined gaze directions while following a predetermined path and
- said at least two values comprise a continuous set of values of said one or more optical features of each ophthalmic lens corresponding to said continuous set of gaze directions, and
in step b), said test lenses are obtained by modifying continuously the optical power of a single test lens with variable optical power from one of said at least two values of one or more optical features determined in step a) to the other.

Alternatively, the change of the optical features between the moment where the optical feature corresponding to the first gaze direction is provided by said test lens and the moment where the optical feature corresponding to the second gaze direction is provided by said test lens may be determined as described in document WO2020/245247.

The speed of change of the optical feature exhibited by the test lens is for example simulating a movement of the eye at a speed of 10 degrees per second. This advantageously corresponds to a speed where most subjects have satisfactory visual performances. The speed may be increased when the subjects indicates that an increased speed allows distinguishing more easily between the test lenses.

Thanks to this dynamic embodiment, realistic simulation may be provided to the eyes of the subject, as the subject may look at his current real environment through the test lens and experience the change in perception that he will have when actually moving his eyes while using the ophthalmic lenses compared.

Thanks to this dynamic embodiment, the subject may compare his perception through the two ophthalmic lenses as if he was gazing at a moving visual target moving continuously in his environment. Thus, the subject may in particular experience the optical aberrations introduced by each of said two ophthalmic lenses in the periphery of the lens. The eyes of the subject do not move, but the optical features of the test lens are modified continuously accordingly. The continuous change in the optical features of the test lens simulates the movement of the eyes looking through different parts of one of the two ophthalmic lenses.

The parameters of this dynamic simulation: path of the eyes between the first and second gaze directions, speed of the change of the optical features, the angle A..., may be predetermined and the same for every subject, or may be customized for each subject. A speed of change of the optical power of said single test lens or said path may be determined taking into account the subject's age and/or sensitivity to a variation of an optical feature of the test lens and/or taking into account a refraction value of the eye of the subject.

In yet another embodiment, it is possible to combine static and dynamic embodiments by providing the eye of the subject with a simulation of both ocular saccades of the subject and smooth movement of the eyes along a continuous path for each of said two ophthalmic lenses.

The parameters of said static or dynamic simulation may correspond to a far vision, intermediate vision or near vision situation.

### Step c)

In step c), the subject observes (block 500 of figure 1) a visual target 5 through each of said test lenses.

Preferably, the subject gazes at the same visual target 5 while the different values of the optical features of the lens determined at step b) are provided.

Preferably, during steps b), c) and d) of the method, the eye of the subject is fixed, not moving. The gaze direction of the subject remains the same. It goes through the center of the test lens. The test lens is here the phoropter lens.

Said visual target 5 may be fixed, not moving.

Said fixed target is preferably placed in front of the subject, so that the gaze direction of the eye goes through the optical center of each test lens.

Said visual target 5 may be placed at a far vision distance from the eye of the subject, at an intermediate distance or at a near vision distance.

Even if the eye of the subject is focused during step c) on a visual target located at a distance from the eyes corresponding to a far vision situation, the optical features of the test lens provided may correspond to an intermediate or near vision.

Alternatively, the visual target may be placed at a distance corresponding to the visual situation of the simulation.

The visual target may comprise any kind of optotype.

The visual target 5 may be the usual environment of the subject itself. The phoropter 2 may then be used as test eyeglasses through which the subject sees his environment. No simulated image is shown to the subject.

In a variant, said visual target may be mobile along the gaze direction of the eye of the subject. The eye of the subject then remains fixed, but the visual target moves away or towards the subject along the gaze direction.

### Step d)

In step d), the subject compares (block 600 of figure 1) his perception of the visual target 5 through the test lenses corresponding to each ophthalmic lens of said two ophthalmic lenses.

He may therefore perceive the difference between said two ophthalmic lenses in a natural environment and natural viewing conditions. He may then compare the lenses, providing for example the same spherical and cylindrical power with and without aspherization or with and without a tint.

In an embodiment of said method, other steps may be performed before step a) according to the invention. The following preliminary steps may be performed:
- determining (block 100 of figure 1) the refractive features of the eye of the subject and
- determining (block 200 of figure 1) said two ophthalmic lenses based on these refractive features.

Said refractive features of the eye of the subject may be determined in far vision conditions, in near vision and/or intermediary vision conditions.

In practice, in said preliminary steps before step a), a spherical and/or cylindrical refraction value of the eye of the subject is determined and said spherical and/or cylindrical power provided by said different optical designs of said ophthalmic lenses is determined based on this spherical and/or cylindrical refraction value of the eye of the subject.

Preferably, the spherical and/or cylindrical refraction value of the eye of the subject is determined by a subjective refraction test allowing less than 0.25 diopter error. For example, the spherical and/or cylindrical refraction value of the eye is determined with an error of 0.01 diopter. Such subjective refraction tests are well-known from the man skilled in the art and will not be described in detail here.

Said optical design of each of said two ophthalmic lenses is calculated based on said spherical and/or cylindrical refraction of the eye of the subject and based on two different predetermined optical design models.

For example, one of said two different predetermined optical design models is with aspherization and the other without, or one of said two different predetermined models is with a tint and the other is clear.

The method according to the invention is performed thanks to the optometry system 1 mentioned above.

Said computer of said device 2 may be integrated to the phoropter 3. It may also control said phoropter 3. In particular, it may control the lenses with variable power of said phoropter 3.

The phoropter 3 of the optometry system 1 used in the method according to the invention comprises a binocular refraction test unit 10 with a first optical refraction element 11 adapted to provide different vision correction powers along a first optical axis OA1 and a second optical refraction element 12 adapted to provide different vision correction powers along a second optical axis OA2 (figure 2).

Said first optical refraction element 11 is configured for providing the first eye of the subject with a first correction power and said second optical refraction element 12 is configured for providing the second eye of the subject with a second correction power.

By correction power, it is meant a dioptric power allowing correcting a refraction error of the eye of the subject, such as spherical power, cylindrical power and axis, prismatic power...

Each of the first and second optical refraction elements 11, 12 comprises a lens, a mirror or a set of such optical components, that has adjustable refractive power features.

In the example shown on the appended figures, and described in the following, each of said first and second optical refraction elements 11, 12 comprises a lens with variable power. It comprises here a deformable liquid lens having an adjustable shape. The optical axis OA1, OA2 mentioned before thus correspond to the optical axis of the corresponding lens.

In addition, the optical refraction element may comprise an ensemble of non-deformable lenses having different optical powers, and a mechanical system that enables to select some of these lenses to group them to form the set of lenses through which the subject can look.

In particular, each optical refraction element 11, 12 may comprise a lens with variable spherical power and an optical component with a variable cylindrical power and variable cylindrical axis. Each optical refraction element 11, 12 then comprises an optical set with a lens with variable spherical power and an optical component with a variable cylindrical power and variable cylindrical axis.

The optical refraction element 11, 12 may also comprise one or more filters, as mentioned earlier, to provide a tint to the test lenses.

Each of the optical refraction elements 11, 12 is intended to be placed in front of one of the eyes of the subject, close to this eye. It is placed, for example, at a distance comprised between 5 and 50 millimeters from the eye.

The optical set has an overall spherical power Sph corresponding to the spherical optical power, expressed in diopters. The cylindrical components of its refractive power are those of an equivalent cylindrical lens that has a cylindrical power Cyl (expressed for instance in diopters), and whose cylinder has an orientation represented by an angle Acyl. Each of the first and second refraction correction, provided by the corresponding optical refraction elements 11, 12, may be characterized by the values of these three refractive power parameters Sph, Cyl and Acyl.

This refractive correction could be equally characterized by the values of any other set of parameters representing the above mentioned refractive power features of the optical sets of the optical refraction elements 11, 12, such as the triplet {M, J0, J45}, where the equivalent sphere M is equal to the spherical power S plus half of the cylindrical power C (M = S+C/2), and where J0 and J45 are the refractive powers of two Jackson crossed cylinders lenses representative of the cylindrical refractive features of the lens or of the set of lenses of the optical refraction elements 11, 12.

Said optical refraction elements 11, 12 are mounted on a common support 13 that extends between the optical refraction elements, above them, along a longitudinal axis that is horizontal.

This support 13 is linked to a global supporting structure 14 that lies on a table or on the ground.

The system 1 according to the invention also comprises a visual target 5. Said visual target 5 may be displayed on an active or passive screen 4, as shown on figure 2. It can also be an element of the environment of the subject.

According to an example of the method of the invention defined in claim the following steps are performed.

A precise refraction value of the eye of the subject is determined using the phoropter 3. This refraction value is obtained thanks to a subjective test using the optical refraction element 11, 12 comprising the lens with variable power of the phoropter 3. The phoropter 3 is programmed to perform this subjective test and deduce the refraction value from the answers of the subject.

The device 2 of said system is programmed to determine two different ophthalmic lenses based on the refraction value of the eye of the subject.

The two ophthalmic lenses are determined according to two different optical designs, for example an optical design comprising an aspherization and an optical design without aspherization. Both ophthalmic lenses may be single vision lenses or progressive lenses.

Alternatively, the two ophthalmic lenses may be determined with slightly different refraction values of the eye of the subject with the same optical design model: both with aspherization or tint or both without aspherization or tint.

The device 2 is programmed to calculate the optical features, such as spherical power, cylindrical power and axis, transmittance for each of the two ophthalmic lenses determined based on the optical design models and the refraction values of the subject for a predetermined set of points of the back and/or front face of the lens.

The device 2 is then programmed to select one or more points of said set of points corresponding to one or more gaze directions going through said points.

For example, two points are selected: one is the center of the lens and the other a peripheral point of the lens. They correspond to a gaze direction going through the center of the lens and a gaze direction going through the peripheral point of the lens.

The device 2 is programmed to calculate the expected value of the refractive power of each of said two ophthalmic lenses along said gaze directions for each of said two ophthalmic lenses.

The optical refraction element 11, 12 form here the test lenses used in the method according to the invention. The phoropter 3 is programmed to alternatively set the power of the optical refraction element 11, 12 placed in front of the corresponding eye of the subject to provide said expected value of the refractive power for each gaze directions and each ophthalmic lens while the subject is looking straight through the optical refraction element. The visual target 5 is fixed, placed in front of the phoropter, straight ahead from the subject's eye and the corresponding test lenses formed by the optical refraction element 11, 12.

The eye of the subject remains still, focused on said visual target 5 while the phoropter 3 provides alternatively the different test lenses formed by the optical refraction element 11, 12 controlled to exhibit different optical features.

The gaze direction of the subject remains straight ahead, going through the center of each test lens.

The phoropter 3 or eye care practitioner then asks the subject which of the test lenses, and therefore which of the two ophthalmic lenses provides the best visual performance and/or comfort.

The subject assesses his visual performance with each test lens. The preferred ophthalmic lens is deduced from this assessment.

The phoropter or eye care practitioner recommends the ophthalmic lens preferred by the subject (block 700 of figure 1).

Thanks to the method and device according to the invention, the subject may compare two ophthalmic lenses not only by visualizing the effect of each ophthalmic lens looked through at its center, but also the effect of each ophthalmic lens when the subject looks through other parts of the ophthalmic lens.

The subject may therefore assess his visual performance with each lens more precisely. The determination of the ophthalmic lens best adapted to the subject is improved.

## Claims

1. Method for allowing a subjective comparison of two ophthalmic lenses having different optical designs by an eye of a subject, said method comprising:
a) determining (300) two values of one or more optical features of each ophthalmic lens of said two ophthalmic lenses, each value being determined along one of two predetermined gaze directions (G1, G2) of the eye of the subject through said ophthalmic lens, the two predetermined gaze directions (G1, G2) being separated by a predetermined angle (A),
**characterised by**
b) for each ophthalmic lens of said two ophthalmic lenses, placing (400) successively in front of said eye of the subject test lenses, each of these test lenses exhibiting optical features having one of said two values of one or more optical features determined in step a) for this ophthalmic lens,
c) observing (500) a target (5) through each of said test lenses,
d) comparing (600) the perception by said eye of said subject of this target through the test lenses corresponding to each ophthalmic lens of said two ophthalmic lenses.

2. Method according to claim 1, wherein one of said two predetermined gaze directions goes through the optical center of the ophthalmic lens, and the other goes through an off-center point separated from the optical center by said predetermined angle.

3. Method according to any one of claims 1 and 2, wherein said one or more optical features comprises one or more of the following: spherical power, cylindrical power and axis, prism power, transmittance at a given wavelength or within a wavelength range.

4. Method according to anyone of claims 1 to 3, wherein said predetermined angle (A) separating the two predetermined gaze directions is determined taking into account a refraction value of the eye of the subject and/or a sensitivity of the eye of the subject to a variation of said optical features of the ophthalmic lens.

5. Method according to any one of claims 1 to 4, wherein in step a), said two predetermined gaze directions of the eye of the subject through said ophthalmic lens are comprised in a predetermined discrete number of gaze directions and in step b), a corresponding predetermined discrete number of test lenses is placed in front of the eye of the subject.

6. Method according to claim 5, wherein in step b), said test lenses are obtained by modifying the optical power of a single test lens with variable optical power in order for said single test lens to exhibit successively each value of said two values of one or more optical features determined in step a).

7. Method according to any one of claims 1 to 6, wherein in step a):
- said two predetermined gaze directions of the eye of the subject through said ophthalmic lens are comprised in a continuous set of gaze directions corresponding to the gaze directions of the eye of the subject moving continuously between said two predetermined gaze directions while following a predetermined path and
- said two values are comprised in a continuous set of values of said one or more optical features of each ophthalmic lens corresponding to said continuous set of gaze directions, and
in step b), said test lenses are obtained by modifying continuously the optical power of a single test lens with variable optical power from one of said two values of one or more optical features determined in step a) to the other.

8. Method according to any one of claims 1 to 7, wherein in step b), a speed of change of the optical power of said single test lens is determined taking into account the subject's age and/or sensitivity to a variation of an optical feature of the test lens and/or taking into account a refraction value of the eye of the subject.

9. Method according to any one of claims 1 to 8, wherein, in step c), the eye of the subject gazes at a fixed target placed in front of it, so that the gaze direction of the eye goes through the optical center of each test lens.

10. Method according to claim 9, wherein said target is placed at a far vision distance from the eye of the subject, at an intermediate distance or at a near vision distance.

11. Method according to any one of claims 1 to 10, wherein, in a preliminary step before step a), a spherical and/or cylindrical refraction value of the eye of the subject is determined and said spherical and/or cylindrical power provided by said different optical designs of said ophthalmic lenses is determined based on this spherical and/or cylindrical refraction value of the eye of the subject.

12. Method according to claim 11, wherein in said preliminary step, the spherical and/or cylindrical refraction value of the eye of the subject is determined by a subjective refraction test allowing less than 0.25 diopter error.

13. Method according to any one of claims 11 to 12, wherein, in said preliminary step, said optical designs of said ophthalmic lenses are calculated based on said spherical and/or cylindrical refraction of the eye of the subject and based on two different predetermined models, and:
- one of said two different predetermined models is with aspherization and the other without, or
- one of said two different predetermined models is with a tint and the other is clear.

14. Method according to any one of claims 1 to 13, wherein said values of one or more optical features of each ophthalmic lens are determined taking into account the fitting parameters of the ophthalmic lens on the head of the subject and/or the sensitivity of the subject to a variation of said optical feature.

15. System (1) for allowing an eye of a subject to compare two ophthalmic lenses having different optical designs, said system (1) comprising:
- a device (2) programmed for determining two values of one or more optical features of each ophthalmic lens of said two ophthalmic lenses, each value being determined along one of two predetermined gaze directions of the eye of the subject through said ophthalmic lens, the two predetermined gaze directions being separated by a predetermined angle,
**characterised by** further comprising
- a phoropter (3) controlled for providing test lenses (11, 12), each of these test lenses exhibiting optical features having one of said two values of one or more optical features determined by said device,
- a target (5) placed in front of said test lenses provided by said phoropter.

## Patentansprüche

1. Verfahren zum Erlauben eines subjektiven Vergleichs von zwei ophthalmischen Linsen, die unterschiedliche optische Auslegungen aufweisen, mittels eines Auges einer Person, wobei das Verfahren umfasst:
a) Ermitteln (300) von zwei Werten von einem oder mehreren optischen Merkmalen von jeder ophthalmischen Linse der beiden ophthalmischen Linsen, wobei jeder Wert entlang einer von zwei vorbestimmten Blickrichtungen (G1, G2) des Auges der Person durch die ophthalmische Linse ermittelt wird, wobei die beiden vorbestimmten Blickrichtungen (G1, G2) um einen vorbestimmten Winkel (A) voneinander getrennt sind,
**gekennzeichnet durch**
b) für jede ophthalmische Linse der beiden ophthalmischen Linsen, nacheinander Platzieren (400) von Testlinsen vor dem Auge der Person, wobei jede dieser Testlinsen optische Merkmale vorweist, die einen der beiden Werte des einen oder der mehreren optischen Merkmale aufweist, die im Schritt a) für diese Linse ermittelt wurden,
c) Beobachten (500) eines Ziels (5) durch jede der Testlinsen,
d) Vergleichen (600) der Wahrnehmung mittels des Auges der Person dieses Ziels durch die Testlinsen, die jeder ophthalmischen Linse der beiden ophthalmischen Linsen entsprechen.

2. Verfahren nach Anspruch 1, wobei eine der beiden Blickrichtungen den optischen Mittelpunkt der ophthalmischen Linse durchquert und wobei die andere einen außermittigen Punkt durchquert, der um den vorbestimmten Winkel von dem optischen Mittelpunkt getrennt ist.

3. Verfahren nach einem der Ansprüche 1 und 2, wobei das eine oder die mehreren optischen Merkmale eines oder mehrerer umfasst von: einer sphärischen Brechkraft, einer zylindrischen Brechkraft und Achse, einer Prismenbrechkraft, einem Transmissionsgrad bei einer gegebenen Wellenlänge oder innerhalb eines Wellenlängenbereichs.

4. Verfahren nach einem der Ansprüche 1 bis 3, wobei der vorbestimmte Winkel (A), der die beiden vorbestimmten Blickrichtungen trennt, unter Berücksichtigung eines Refraktionswerts des Auges der Person und/oder einer Empfindlichkeit des Auges der Person für eine Variation der optischen Merkmale der ophthalmischen Linse ermittelt wird.

5. Verfahren nach einem der Ansprüche 1 bis 4, wobei im Schritt a) die beiden vorbestimmten Blickrichtungen des Auges der Person durch die ophthalmische Linse in einer vorbestimmten diskreten Anzahl von Blickrichtungen enthalten sind und im Schritt b) eine entsprechende diskrete Anzahl von Testlinsen vor dem Auge der Person platziert wird.

6. Verfahren nach Anspruch 5, wobei im Schritt b) die Testlinsen erhalten werden, indem die Brechkraft einer einzelnen Testlinse mit einer variablen Brechkraft modifiziert wird, damit die einzelne Testlinse nacheinander jeden Wert der beiden Werte von einem oder mehreren optischen Merkmalen vorweist, die im Schritt a) ermittelt wurden.

7. Verfahren nach einem der Ansprüche 1 bis 6, wobei im Schritt a):
- die beiden vorbestimmten Blickrichtungen des Auges der Person durch die ophthalmische Linse in einem kontinuierlichen Satz von Blickrichtungen enthalten sind, der den Blickrichtungen des Auges der Person entspricht, das sich kontinuierlich zwischen den beiden vorbestimmten Blickrichtungen bewegt, während es einem vorbestimmten Weg folgt, und
- die beiden Werte in einem kontinuierlichen Satz von Werten des einen oder der mehreren optischen Merkmale von jeder ophthalmischen Linse enthalten sind, der dem kontinuierlichen Satz von Blickrichtungen entspricht, und
wobei im Schritt b) die Testlinsen erhalten werden, indem die Brechkraft einer einzelnen Testlinse kontinuierlich mit einer variablen Brechkraft von einem der beiden Werte von einem oder mehreren optischen Merkmalen, die im Schritt a) ermittelt wurden, zu dem anderen modifiziert wird.

8. Verfahren nach einem der Ansprüche 1 bis 7, wobei im Schritt b) eine Geschwindigkeit einer Änderung der Brechkraft der einzelnen Testlinse unter Berücksichtigung des Alters der Person und/oder einer Empfindlichkeit für eine Variation eines optischen Merkmals der Testlinse und/oder unter Berücksichtigung eines Refraktionswerts des Auges der Person ermittelt wird.

9. Verfahren nach einem der Ansprüche 1 bis 8, wobei im Schritt c) das Auge der Person auf ein festes Ziel blickt, das vor ihm platziert wird, sodass die Blickrichtung des Auges den optischen Mittelpunkt der Testlinse durchquert.

10. Verfahren nach Anspruch 9, wobei das Ziel in einem Fernsichtabstand von dem Auge der Person, einem Zwischenabstand oder in einem Nahsichtabstand platziert wird.

11. Verfahren nach einem der Ansprüche 1 bis 10, wobei in einem vorbereitenden Schritt vor dem Schritt a) ein sphärischer und/oder ein zylindrischer Refraktionswert des Auges der Person ermittelt wird und wobei die sphärische und/oder zylindrische Brechkraft, die mittels der unterschiedlichen optischen Auslegungen der ophthalmischen Linsen bereitgestellt werden, basierend auf diesem sphärischen und/oder zylindrischen Refraktionswert des Auges der Person ermittelt wird.

12. Verfahren nach Anspruch 11, wobei in dem vorbereitenden Schritt der sphärische und/oder der zylindrische Refraktionswert des Auges der Person mittels eines subjektiven Refraktionstests ermittelt wird, der einen Fehler von weniger als 0,25 Dioptrien erlaubt.

13. Verfahren nach einem der Ansprüche 11 bis 12, wobei in dem vorbereitenden Schritt die optischen Auslegungen der ophthalmischen Linsen basierend auf der sphärischen und/oder zylindrischen Refraktion des Auges der Person und basierend auf zwei unterschiedlichen vorbestimmten Modellen berechnet werden, und wobei:
- eines der beiden unterschiedlichen vorbestimmten Modelle eine Asphärisierung aufweist und das andere nicht, oder
- eines der beiden unterschiedlichen vorbestimmten Modelle eine Färbung aufweist und das andere klar ist.

14. Verfahren nach einem der Ansprüche 1 bis 13, wobei die Werte des einen oder der mehreren optischen Merkmale von jeder ophthalmischen Linse unter Berücksichtigung der Anpassungsparameter der ophthalmischen Linse an dem Kopf der Person und/oder der Empfindlichkeit der Person für eine Variation des optischen Merkmals ermittelt werden.

15. System (1) zum Erlauben, dass ein Auge einer Person zwei ophthalmische Linsen vergleicht, die unterschiedliche optische Auslegungen aufweisen, wobei das System (1) umfasst:
- eine Vorrichtung (2), die programmiert ist zum Ermitteln von zwei Werten von einem oder mehreren optischen Merkmalen von jeder ophthalmischen Linse der beiden ophthalmischen Linsen, wobei jeder Wert entlang einer von zwei vorbestimmten Blickrichtungen des Auges der Person durch die ophthalmische Linse ermittelt wird, wobei die beiden vorbestimmten Blickrichtungen durch einen vorbestimmten Winkel voneinander getrennt sind, **dadurch gekennzeichnet, dass** es ferner umfasst:
- einen Phoropter (3), der zum Bereitstellen von Testlinsen (11, 12) gesteuert wird, wobei jede dieser Testlinsen optische Merkmale vorweist, die einen der beiden Werte von einem oder mehreren optischen Merkmalen aufweist, die durch die Vorrichtung ermittelt werden,
- ein Ziel (5), das vor den Testlinsen platziert wird, die von dem Phoropter bereitgestellt werden.

## Revendications

1. Procédé pour permettre une comparaison subjective de deux lentilles ophtalmiques ayant des conceptions optiques différentes par l'œil d'un sujet, ledit procédé comprenant les étapes suivantes :
a) déterminer (300) deux valeurs d'une ou plusieurs caractéristiques optiques de chaque lentille ophtalmique desdites deux lentilles ophtalmiques, chaque valeur étant déterminée le long d'une des deux directions prédéterminées (G1, G2) du regard de l'œil du sujet à travers ladite lentille ophtalmique, les deux directions prédéterminées (G1, G2) du regard étant séparées par un angle prédéterminé (A),
**caractérisé par** les étapes suivantes
b) pour chaque lentille ophtalmique desdites deux lentilles ophtalmiques, placer (400) successivement devant ledit œil des lentilles d'essai du sujet, chacune de ces lentilles d'essai présentant des caractéristiques optiques ayant l'une desdites deux valeurs d'une ou plusieurs caractéristiques optiques déterminées à l'étape a) pour cette lentille ophtalmique,
c) observer (500) une cible (5) à travers chacune desdites lentilles d'essai,
d) comparer (600) la perception de cette cible par ledit œil dudit sujet à travers les lentilles d'essai correspondant à chaque lentille ophtalmique desdites deux lentilles ophtalmiques.

2. Procédé selon la revendication 1, dans lequel l'une desdites deux directions de regard prédéterminées passe par le centre optique de la lentille ophtalmique, et l'autre passe par un point excentré séparé du centre optique dudit angle prédéterminé.

3. Procédé selon l'une quelconque des revendications 1 et 2, dans lequel lesdites une ou plusieurs caractéristiques optiques comprennent une ou plusieurs des caractéristiques suivantes : la puissance sphérique, la puissance et l'axe cylindriques, la puissance prismatique, la transmittance à une longueur d'onde donnée ou dans une plage de longueurs d'onde.

4. Procédé selon l'une quelconque des revendications 1 à 3, dans lequel ledit angle prédéterminé (A) séparant les deux directions prédéterminées du regard est déterminé en tenant compte d'une valeur de réfraction de l'œil du sujet et/ou d'une sensibilité de l'œil du sujet à une variation desdites caractéristiques optiques de la lentille ophtalmique.

5. Procédé selon l'une quelconque des revendications 1 à 4, dans lequel, à l'étape a), lesdites deux directions prédéterminées du regard de l'œil du sujet à travers ladite lentille ophtalmique sont comprises dans un nombre discret prédéterminé de directions du regard et, à l'étape b), un nombre discret prédéterminé correspondant de lentilles d'essai est placé devant l'œil du sujet.

6. Procédé selon la revendication 5, dans lequel à l'étape b), lesdites lentilles d'essai sont obtenues en modifiant la puissance optique d'une lentille d'essai unique à puissance optique variable afin que ladite lentille d'essai unique présente successivement chaque valeur desdites deux valeurs d'une ou plusieurs caractéristiques optiques déterminées à l'étape a).

7. Procédé selon l'une quelconque des revendications 1 à 6, dans lequel, à l'étape a) :
- lesdites deux directions de regard prédéterminées de l'œil du sujet à travers ladite lentille ophtalmique sont comprises dans un ensemble continu de directions de regard correspondant aux directions de regard de l'œil du sujet se déplaçant continuellement entre lesdites deux directions de regard prédéterminées tout en suivant un trajet prédéterminé, et
- lesdites deux valeurs sont comprises dans un ensemble continu de valeurs desdites une ou plusieurs caractéristiques optiques de chaque lentille ophtalmique correspondant audit ensemble continu de directions du regard, et
à l'étape b), lesdites lentilles d'essai sont obtenues en modifiant en continu la puissance optique d'une seule lentille d'essai à puissance optique variable, d'une desdites deux valeurs d'une ou plusieurs caractéristiques optiques déterminées à l'étape a) à l'autre.

8. Procédé selon l'une quelconque des revendications 1 à 7, dans lequel, à l'étape b), la vitesse de changement de la puissance optique de ladite lentille d'essai unique est déterminée en tenant compte de l'âge du sujet et/ou de sa sensibilité à une variation d'une caractéristique optique de la lentille d'essai et/ou en tenant compte d'une valeur de réfraction de l'œil du sujet.

9. Procédé selon l'une quelconque des revendications 1 à 8, dans lequel, à l'étape c), l'œil du sujet regarde une cible fixe placée devant lui, de sorte que la direction du regard de l'œil passe par le centre optique de chaque lentille d'essai.

10. Procédé selon la revendication 9, dans lequel ladite cible est placée à une distance de vision de loin de l'œil du sujet, à une distance intermédiaire ou à une distance de vision de près.

11. Procédé selon l'une quelconque des revendications 1 à 10, dans lequel, dans une étape préliminaire précédant l'étape a), une valeur de réfraction sphérique et/ou cylindrique de l'œil du sujet est déterminée et ladite puissance sphérique et/ou cylindrique fournie par lesdites différentes conceptions optiques desdites lentilles ophtalmiques est déterminée sur la base de cette valeur de réfraction sphérique et/ou cylindrique de l'œil du sujet.

12. Procédé selon la revendication 11, dans lequel, dans ladite étape préliminaire, la valeur de réfraction sphérique et/ou cylindrique de l'œil du sujet est déterminée par un test de réfraction subjectif permettant une erreur de moins de 0,25 dioptrie.

13. Procédé selon l'une quelconque des revendications 11 et 12, dans lequel, dans ladite étape préliminaire, lesdites conceptions optiques desdites lentilles ophtalmiques sont calculées sur la base de ladite réfraction sphérique et/ou cylindrique de l'œil du sujet et sur la base de deux modèles prédéterminés différents, et :
- un desdits deux modèles prédéterminés différents est avec asphérisation et l'autre sans, ou
- un desdits deux modèles prédéterminés différents est avec une teinte et l'autre est clair.

14. Procédé selon l'une quelconque des revendications 1 à 13, dans lequel lesdites valeurs d'une ou plusieurs caractéristiques optiques de chaque lentille ophtalmique sont déterminées en tenant compte des paramètres d'ajustement des lentilles ophtalmiques sur la tête du sujet et/ou de la sensibilité du sujet à une variation de ladite caractéristique optique.

15. Système (1) permettant à l'œil d'un sujet de comparer deux lentilles ophtalmiques ayant des conceptions optiques différentes, ledit système (1) comprenant :
- un dispositif (2) programmé pour déterminer deux valeurs d'une ou plusieurs caractéristiques optiques de chaque lentille ophtalmique desdites deux lentilles ophtalmiques, chaque valeur étant déterminée le long d'une de deux directions prédéterminées du regard de l'œil du sujet à travers ladite lentille ophtalmique, les deux directions prédéterminées du regard étant séparées par un angle prédéterminé,
**caractérisé en ce qu'**il comprend en outre :
- un réfracteur (3) commandé pour fournir des lentilles d'essai (11, 12), chacune de ces lentilles d'essai présentant des caractéristiques optiques ayant l'une desdites deux valeurs d'une ou - plusieurs caractéristiques optiques déterminées par ledit dispositif,
- une cible (5) placée devant lesdites lentilles d'essai fournies par ledit réfracteur.
